# EUROPEAN PATENT SPECIFICATION

(11) **EP 3 397 335 B1**
(45) Date of publication and mention of the grant of the patent: **03.08.2022**
(21) Application number: 16882581.8
(22) Date of filing: 28.12.2016
(51) Int. Cl.: A61N 1/04, A61N 1/24, A61N 1/30, A61N 1/32, A61N 1/40, C12N 13/00, H02M 3/335

(54) **ELECTROPORATION DEVICE WITH IMPROVED SIGNAL GENERATOR**
ELEKTROPORATIONSVORRICHTUNG MIT VERBESSERTEM SIGNALGENERATOR
DISPOSITIF D'ÉLECTROPORATION À GÉNÉRATEUR DE SIGNAUX AMÉLIORÉ

(30) Priority: 28.12.2015 US 201562271955 P
(43) Date of publication of application: 07.11.2018
(73) Proprietor: Inovio Pharmaceuticals, Inc., Plymouth Meeting, PA 19462 (US)
(72) Inventor: STADELMANN, Beat, Plymouth Meeting, PA 19462 (US)
(74) Representative: Leonard, Thomas Charles
(86) International application number: PCT/US2016/068940
(87) International publication number: WO 2017/117251

(56) References cited:
- WO-A1-2006/126904
- US-A- 6 118 679
- US-A1- 2002 068 338
- US-A1- 2002 068 338
- US-A1- 2008 312 579
- US-A1- 2011 140 663
- US-A1- 2011 140 663

## Description

### BACKGROUND

The present disclosure generally relates to an electroporation device having an improved signal generator for generating high voltage electroporation signals. The invention is set out in the appended claims.

### SUMMARY

Medical devices, such as electroporation devices, require high voltage generators to generate the necessary supply of energy. During the electroporation process, electrodes in contact with the target tissue require electrical power be delivered at a particular voltage and amperage in order to produce the desired electroporation effects (e.g., 200 V at 0.5 Amps). Generally speaking, the high voltage levels required during the electroporation process require a voltage generator that includes a number of high-capacity capacitors. These capacitors, in turn, are very bulky in physical size and require relatively long periods of time to charge before the electroporation process may begin. These attributes are burdensome in handheld units where size and weight are to be kept at a minimum. Furthermore, long charging times can hamper the user's ability to administer the electroporation treatment in a timely and accurate manner. Still further, capacitor-based systems suffer from signal degradation over time

The disclosure provides a signal generator that generates a plurality of lower voltages and combines them in series to create a high voltage.

In one aspect, a handset for use in an electroporation device, the handset including a housing, and a signal amplifier positioned within the housing. Where the signal amplifier includes a primary winding, a plurality of secondary windings coupled together in a series configuration, where a storage capacitor and a fly-back diode are coupled to each of the plurality of secondary windings, and an array having a plurality of electrodes in electrical communication with the signal amplifier.

In another aspect, an electroporation device including a housing, and a signal generator positioned within the housing. The signal generator including a signal amplifier having a primary winding and a plurality of secondary windings coupled together in a series configuration, where a storage capacitor and a fly-back diode are coupled to each of the plurality of secondary windings, a power supply, and a power switch configured to supply a voltage from the power supply across the primary winding, and an array having one or more electrodes in electrical communication with the signal generator.

In still another aspect, an electroporation system including a base station, and a handset removably coupled to the base station. The handset including a housing, an injection assembly, a power supply, and a signal generator positioned within the housing of the handset and in operable communication with the injection assembly. The signal generator including a signal amplifier having a primary winding, and a plurality of secondary windings coupled together in a series configuration, where a storage capacitor and a fly-back diode are coupled to each of the plurality of secondary windings, and a power switch configured to supply a voltage from the power supply across the primary winding, and an array having at least one electrode extending therefrom and in electrical communication with the signal generator. US 2008/312579 A1 discloses an electroporation device with a galvanic-isolated current driver.

### BRIEF DESCRIPTION OF THE DRAWINGS

FIG. 1 is a schematic view of an electroporation device showing a handset and a base unit in a docked configuration.
FIG. 2 is a diagram of the voltage amplifier of FIG. 1, in accordance with some embodiments.
FIG. 3 is a block diagram of the signal generator and power supply of FIG. 1, in accordance with some embodiments.

### DETAILED DESCRIPTION

Before any embodiments of the disclosure are explained in detail, it is to be understood that the disclosure is not limited in its application to the details of construction and the arrangement of components set forth in the following description or illustrated in the following drawings. The disclosure is capable of other embodiments and of being practiced or of being carried out in various ways.

It should also be noted that a plurality of other structural components may be utilized to implement the disclosure. Furthermore, and as described in subsequent paragraphs, the specific configurations illustrated in the drawings are intended to exemplify embodiments of the disclosure. Alternative configurations are possible.

"Agent" may mean a polypeptide, a polynucleotide, a small molecule, or any combination thereof. The agent may be a recombinant nucleic acid sequence encoding an antibody, a fragment thereof, a variant thereof, or a combination thereof, as detailed in PCT/US2014/070188. "Agent" may mean a composition comprising a polypeptide, a polynucleotide, a small molecule, or any combination thereof. The composition may comprise a recombinant nucleic acid sequence encoding an antibody, a fragment thereof, a variant thereof, or a combination thereof, as detailed in PCT/US2014/070188. The agent may be formulated in water or a buffer, for example. The buffer may be saline-sodium citrate (SSC) or phosphate-buffered saline (PBS), for example. The ionic content of the buffers may increase conductivity, resulting in increased current flow in the targeted tissue. The concentration of the formulated polynucleotide may be between 1 µg and 20 mg/ml. The concentration of the formulated polynucleotide may be 1µg/ml, 10µg/ml, 25µg/ml, 50µg/ml, 100µg/ml, 250µg/ml, 500µg/ml, 750µg/ml, 1mg/ml, 10mg/ml, 15mg/ml, or 20mg/ml, for example.

A "peptide," "protein," or "polypeptide" as used herein can mean a linked sequence of amino acids and can be natural, synthetic, or a modification or combination of natural and synthetic.

"Polynucleotide" or "oligonucleotide" or "nucleic acid" as used herein means at least two nucleotides covalently linked together. A polynucleotide can be single stranded or double stranded, or can contain portions of both double stranded and single stranded sequence. The polynucleotide can be DNA, both genomic and cDNA, RNA, or a hybrid. The polynucleotide can contain combinations of deoxyribo- and ribo-nucleotides, and combinations of bases including uracil, adenine, thymine, cytosine, guanine, inosine, xanthine hypoxanthine, isocytosine, isoguanine, and synthetic or non-naturally occurring nucleotides and nucleosides. Polynucleotides may be a vector. Polynucleotides can be obtained by chemical synthesis methods or by recombinant methods.

"Vector" as used herein means a nucleic acid sequence containing an origin of replication. A vector can be a viral vector, bacteriophage, bacterial artificial chromosome, or yeast artificial chromosome. A vector can be a DNA or RNA vector. A vector can be a selfreplicating extrachromosomal vector, and preferably, is a DNA plasmid.

The term "electroporation," ("EP") as used herein refers to the use of an electric field pulse to induce reversible microscopic pathways (pores) in a bio-membrane; their presence allows agents to pass from one side of the cellular membrane to the other.

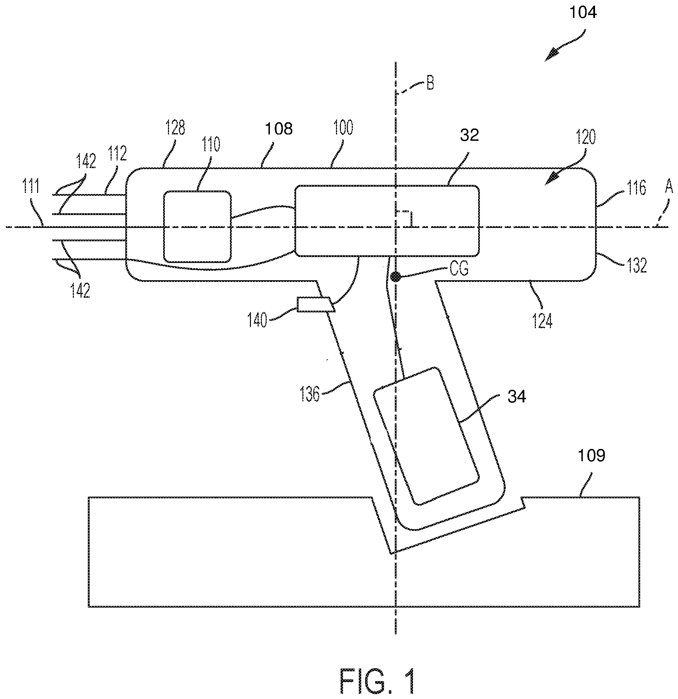

The present disclosure relates to a handset 100 for an electroporation device 104 that includes an improved signal generator 32 for producing a predetermined electroporation signal. Illustrated in FIG. 1, the electroporation device 104 includes a base unit 108, and a handset 100 that may be detachably docked to the base unit 108. The base unit 108 is generally positioned on a table or other flat surface and is in electrical communication with and able to charge the power supply 34 when the handset 100 and the base unit 108 are in a docked or coupled configuration.

Illustrated in FIG. 1, the handset 100 of the electroporation device 104 includes a housing 108, an electrode array 112 coupled to the housing 108, a power supply 34 positioned within the housing 108, and a signal generator 32 in electrical communication with both the power supply 34 and the electrode array 112. The handset 100 also includes an injection assembly 110 to administer agent to the target tissue via a hypodermic needle 111. During use, the electroporation device 100 facilitates the introduction of agent into cells of a target tissue (for example, skin or muscle) of a mammal using electroporation pulses generated by the signal generator 32. The handset 100 requires the generation of very high voltage values (for example, 200 Volts) to generate the electroporation pulses. The handset 100 uses the signal generator 32 to generate the very high voltage values from a power source (for example, Lithium-ion batteries) that supplies a lower voltage value.

Illustrated in Fig. 1, the housing 108 of the handset 100 is formed from two halves or members 116 coupled together to form a volume 120 therebetween. Specifically, the members 116 form a pistol-shape having an upper portion 124 with a front end 128 and a rear end 132, and a handle portion 136 extending from the upper 124 to form a distal end. In some embodiments, the handle portion 136 may also include a trigger 140 or other user input to allow the user to dictate the administration of the electroporation signal to the target tissue. While the housing 108 of the handset 100 is illustrated in a pistol-shape, it is to be understood that the housing 108 may include additional shapes or accommodate different grip styles.

The electrode array 112 includes a plurality of electrodes 142 each extending outwardly from the front end 128 of the upper portion 124 of the housing 108. Each electrode 142 is in electrical communication with the signal generator 32 and is configured to relay the electroporation signal to the target tissue during operation of the device 104.

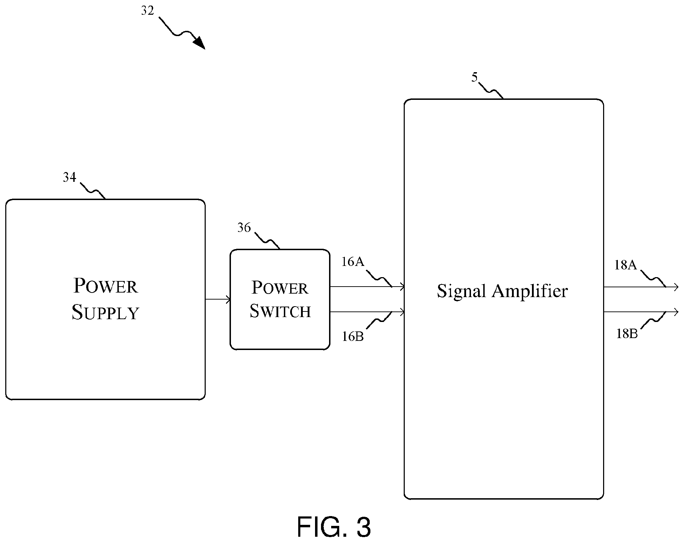

FIGS. 1 and 3 illustrate a signal generator 32. The signal generator 32 includes, among other components, a power switch 36, and a voltage amplifier 5. In the illustrated embodiment, the signal generator 32 is positioned within the housing 108 such that the overall center of gravity (CG) of the handset 100 is positioned proximate the intersection of the handle portion 136 and the upper portion 124. In some embodiments, the upper portion 124 of the housing 108 may define an axis A extending longitudinally therethrough such that an axis B positioned perpendicular to the axis A and passing through the center of gravity (CG) also passes through the handle portion 136 of the housing 108 (see FIG. 1).

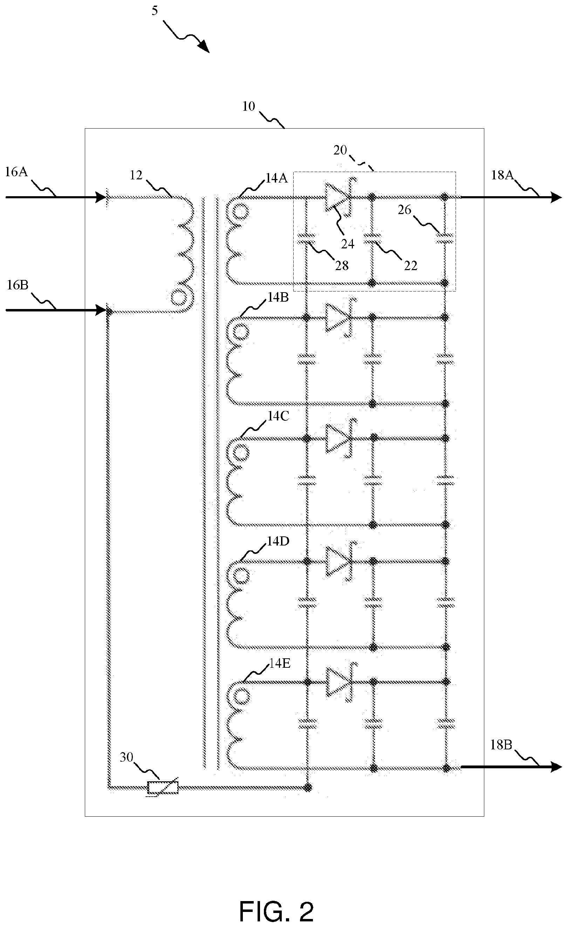

FIG. 2 illustrates the voltage amplifier 5 including, among other components, an amplifier housing 10, a primary winding 12 and a plurality of secondary windings 14A-E. The primary winding 12 and the plurality of secondary windings 14A-E are disposed within the amplifier housing 10. In the embodiment illustrated in FIG. 1, the plurality of secondary windings 14A-E includes five secondary windings. In other embodiments, the plurality of secondary windings 14A-E can include more or less secondary windings. Also, in other embodiments, the voltage amplifier 5 can include more than one primary winding. First and second inputs 16A-B provide connections between the primary windings 12 and one or more components that are external to the amplifier housing 10. The voltage across the primary winding 12 is equal to a voltage difference between the first and second inputs 16A-B. The voltage across each secondary winding is equal to the voltage across the primary winding 12 multiplied by a turn ratio. The turn ratio is the ratio of the number of turns of the primary winding and the number of turns of a secondary winding. For example, if the number of turns of the primary winding 12 is five and the number of turns of secondary winding 14A is five, then the voltage across the secondary winding 14A is equal to the voltage across the primary winding 12 (i.e., turn ratio is 1:1).

In the embodiment illustrated in FIG. 2, the turn ratio between the primary winding 12 and each of the plurality of secondary windings 14A-E is 1:1. For example, when the voltage across the primary winding 12 is 20 volts, the voltages across each of the plurality of secondary windings 14A-E are also 20 volts. When the plurality of secondary windings 14A-E are connected in series, as illustrated in FIG. 2, the voltage across the plurality of secondary windings 14A-E is equal the sum of voltages across each of the secondary windings. For example, when the voltage across the primary winding 12 is 20 volts, the voltage across the plurality of secondary windings 14A-E is 100 volts (as a result of five secondary windings).

First and second outputs 18A-B provide connections between the plurality of secondary windings 14A-E and one or more components that are external to the housing 10. The voltage across the plurality of secondary windings 14A-E is equal to a voltage difference between the first and second outputs 18A-B. For example, when the voltage across the plurality of secondary windings 14A-E is 100 volts, the voltage difference between the first and second outputs 18A-B is 100 volts.

A plurality of electrical components 20 can be coupled to each of the plurality of secondary windings 14A-E. The plurality of electrical components 20 includes, among other components, a storage capacitor 22, a fly-back diode 24, a filtering capacitor 26, and a balancing capacitor 28. In some embodiments, the plurality of electrical components 20 are configured as illustrated in FIG. 2 and discussed below. In some embodiments, the storage capacitor 22 and the filtering capacitor 26 are coupled to each other in a parallel configuration. Also, in some embodiments, the fly-back diode 24 is coupled in a series configuration with the storage capacitor 22 and the filtering capacitor 26. In addition, in some embodiments, the series configuration of the fly-back diode 24 and the storage capacitor 22 are coupled in a parallel configuration with the balancing capacitor 28 and one of the plurality of secondary windings 14A-E.

The plurality of electrical components 20 is disposed within the amplifier housing 10 along with the primary winding 12 and the plurality of secondary windings 14A-E. This configuration enables smaller wire trace lengths between the plurality of electrical components 20 and each of the plurality of secondary windings 14A-E than when compared with components positioned outside of the amplifier housing 10. In addition to permitting a smaller overall footprint for the signal amplifier 5, reducing wire trace lengths reduces the effects of noise (for example, switching noise) on the operation and efficiency of the signal amplifier 5. As such, more accurate and stable electroporation signals may be produced by the handset 100 in a much more compact handset 100.

The above described configuration also enables the use of fewer outputs in the signal amplifier 5. If the plurality of electrical components 20 were outside the amplifier housing 10, each secondary winding would require two outputs. For example, a signal amplifier with five secondary windings would require ten outputs. Disposing the plurality of electrical components 20 within the amplifier housing 10, as illustrated in FIG. 2, enables the use of only two outputs (e.g., the first and second outputs 18A-B). Each additional output requires space and adds to the footprint of a signal amplifier. Thus, by reducing the number of outputs, this configuration enables the signal amplifier 5 to have a smaller footprint and more efficiently utilize space on the corresponding circuit boards and within the volume 120 of the housing 108.

The fly-back diode 24 is necessary to achieve a higher voltage output across the secondary winding 14A than the voltage input across the primary winding 12. The voltage difference between the first and second inputs 16A-B induces a current in the primary winding 12 which creates a magnetic field. The secondary winding 14A picks up the magnetic field and creates a voltage/current spike. Energy from this voltage/current spike is stored in the storage capacitor 22 because the fly-back diode 24 prevents the energy from leaking back into the secondary winding 14A. The energy stored in the storage capacitor 22 can only discharge as a DC voltage output across the secondary winding 14A. The filtering capacitor 26 suppresses voltage spikes across the secondary winding 14A that can occur when the voltage across the secondary winding 14A changes suddenly. The balancing capacitor 28 ensures that the voltages across each of the plurality of secondary windings 14A-E are the same value. Use of the balancing capacitor 28 eliminates the need for snubber circuits in the signal amplifier 5.

The physical size of a capacitor is governed by two factors: working voltage and capacitance. The working voltage is the maximum voltage that the capacitor can operate at. The only way to increase the working voltage of a capacitor is to increase the size of the capacitor. Capacitors with high working voltages are fairly large in physical size. Capacitors with small working voltages are smaller in physical size. Conventional high voltage generators require capacitors with high working voltages. Therefore, conventional high voltage generators tend to be larger in physical size. By generating a plurality of lower voltages and combining them in series to create a high voltage, the signal amplifier 5 is smaller in physical size than conventional high voltage generators because the signal amplifier 5 does not require capacitors with high working voltage. Such attributes are desired in a handset 100 which must be held and maneuvered by the user during use.

Capacitors with high working voltages also require more time to completely charge and discharge. By generating a plurality of lower voltages and combining them in series to create a high voltage, the signal amplifier 5 provides high voltages significantly faster than conventional high voltage generators.

Further, conventional high voltage generators used in medical devices (for example, electroporation pulse generators) that include capacitors with high working voltages present an electrocution safety issue for users of the medical devices. The capacitors in conventional high voltage generators must be charged to high voltages before treatment begins and are capable of producing a high output voltage. During the time in which the capacitors are charged at high voltages but the electroporation pulse has not yet been administered, the capacitors are holding a large amount of electrical energy. This large amount of electrical energy is capable of inflicting serious harm on the users of the medical devices if they are electrocuted by the medical devices. In addition, the large amount of electrical energy can cause conventional high voltage generators to explode. By generating a plurality of lower voltages and combining them in series to create a high voltage, the signal amplifier 5 does not need to store a large amount of electrical energy. Therefore, the electrocution safety issue present in conventional high voltage generators is not present with the signal amplifier 5.

In some embodiments, as illustrated in FIG.1, the signal amplifier 5 includes a thermistor 30. The thermistor is a type of resistor whose resistance is dependent on temperature. In some embodiments, a very small duty cycle is used with the signal amplifier 5. This small duty cycle may be greater than the DC rating of the signal amplifier 5. A control circuit (not shown) can be used to ensure that the signal amplifier 5 does not exceed any component rating by monitoring the thermistor 30. In some embodiments, as illustrated in FIG. 1, the thermistor 30 is coupled between a common node connected to the plurality of secondary windings 14A-E and the primary winding 12.

The power supply 34 supplies a nominal or pulsed DC voltage to the voltage amplifier 5. In the illustrated embodiment, the power supply 34 is powered by one or more batteries or battery packs. In other embodiments, the power supply 34 is powered by mains power having nominal line voltages between, for example, 100V and 240V AC and frequencies of approximately 50-60Hz. In other embodiments, the power supply 34 is powered by a combination of battery power and mains power. In some embodiments, the power supply 34 is powered by USB (i.e., Universal Serial Bus) power having a nominal line voltage of 5V. In some embodiments, the batteries are a type of rechargeable battery. Rechargeable batteries include, for example, lithium-ion, lead-acid, nickel cadmium, nickel metal hydride, etc. Lithium-ion batteries are smaller and lighter than conventional lead-acid batteries.

The power switch 36 regulates the flow of energy from the power supply 34 to signal amplifier 5. The power switch is electrically coupled to the signal amplifier 5 via the first and second inputs 16A-B. The voltage difference between the first and second inputs 16A-B is based on the ON versus OFF time (i.e., the duty cycle) of the power switch 36. In some embodiments, the power switch 36 includes a switching field-effect transistor (FET).

Thus, the disclosure provides, among other things, a voltage amplifier and a signal generator. The invention is set out in the following claims.

## Claims

1. A handset (100) for use in an electroporation device (104), the handset comprising:
a housing (108);
a signal amplifier (5) positioned within the housing, the signal amplifier including: a primary winding (12),
a plurality of secondary windings (14A-14E) coupled together in a series configuration, the plurality of secondary windings having two outputs (18A, 18B), such that a voltage across the plurality of secondary windings is equal to the voltage difference between the two outputs,
wherein a storage capacitor (22) and a fly-back diode (24) are coupled to each of the plurality of secondary windings; and
an array (112) having a plurality of electrodes in electrical communication with the two outputs of the signal amplifier.

2. The handset of claim 1, wherein a turn ratio between the primary winding and each of the plurality of secondary windings is one to one.

3. The handset of claim 1, wherein each fly-back diode and storage capacitor are coupled to a respective one of the plurality of secondary windings in a series configuration.

4. The handset of claim 1, wherein the fly-back diode and the storage capacitor are coupled to each of the plurality of secondary windings in a parallel configuration.

5. The handset of claim 4, wherein a filtering capacitor is coupled in a parallel configuration with the storage capacitor, optionally wherein a balancing capacitor is coupled to each of the plurality of secondary windings in a parallel configuration, further optionally wherein a thermistor is coupled between the plurality of secondary windings and the primary winding.

6. The handset of claim 1, wherein the plurality of secondary windings includes at least five secondary windings.

7. The handset of claim 1, wherein the primary winding, the plurality of secondary windings, the storage capacitor and the fly-back diode are each disposed within a signal generator housing.

8. An electroporation device (104) comprising:
a housing (108);
a signal generator (32) positioned within the housing, the signal generator including:
a signal amplifier (5) having a primary winding (12) and a plurality of secondary windings (14A-14E) coupled together in a series configuration, wherein a storage capacitor (22) and a fly-back diode (24) are coupled to each of the plurality of secondary windings, and the plurality of secondary windings having two outputs (18A, 18B), such that a voltage across the plurality of secondary windings is equal to the voltage difference between the two outputs;
a power supply (34), and
a power switch (36) configured to supply a voltage from the power supply across the primary winding; and
an array (112) having one or more electrodes in electrical communication with the two outputs of the signal generator.

9. The electroporation device of claim 8, wherein a turn ratio between the primary winding and each of the plurality of secondary windings is one to one.

10. The electroporation device of claim 8, wherein the power supply includes a rechargeable battery, optionally wherein the rechargeable battery includes a lithium-ion battery.

11. The electroporation device of claim 8, wherein the fly -back diode and the storage capacitor are coupled to each other in a series configuration, optionally wherein the fly - back diode and the storage capacitor are coupled to each of the plurality of secondary windings in a parallel configuration, further optionally wherein a filtering capacitor is coupled in a parallel configuration with the storage capacitor.

12. The electroporation device of claim 8, wherein a balancing capacitor is coupled to each of the plurality of secondary windings in a parallel configuration.

13. The electroporation device of claim 8, wherein a thermistor is coupled between the plurality of secondary windings and the primary winding.

14. An electroporation system comprising:
a base station (109); and
a handset (100) removably coupled to the base station, the handset including:
a housing (108),
an injection assembly (110) including a hypodermic needle (111) for administering an agent to cells of a target tissue,
a power supply (34),
a signal generator (32) positioned within the housing of the handset and in operable communication with the injection assembly, the signal generator comprising:
a signal amplifier (5) having a primary winding (12), and a plurality of secondary windings (14A-14E) coupled together in a series configuration, wherein a storage capacitor (22) and a fly-back diode (24) are coupled to each of the plurality of secondary windings, and the plurality of secondary windings having two outputs (18A, 18B), such that a voltage across the plurality of secondary windings is equal to the voltage difference between the two outputs, and
a power switch (36) configured to supply a voltage from the power supply across the primary winding, and
an array (112) having at least one electrode (111) extending outwardly from a front end of the housing and in electrical communication with the two outputs of the signal generator, wherein the at least one electrode administers electroporation pulses generated by the signal generator to the cells to induce reversible pores in the cells allowing the agent to pass into the cells.

15. The electroporation system of claim 14, wherein the base station is in electrical communication with the power supply when the base station is coupled to the handset.

## Patentansprüche

1. Handgerät (100) zur Verwendung in einer Elektroporationsvorrichtung (104), wobei das Handgerät Folgendes umfasst:
ein Gehäuse (108);
einen Signalverstärker (5), der in dem Gehäuse positioniert ist, wobei der Signalverstärker Folgendes aufweist:
eine Primärwicklung (12),
eine Vielzahl von Sekundärwicklungen (14A-14E), die in einer Reihenkonfiguration miteinander gekoppelt sind, wobei die Vielzahl von Sekundärwicklungen zwei Ausgänge (18A, 18B) aufweist, so dass eine Spannung über der Vielzahl von Sekundärwicklungen gleich der Spannungsdifferenz zwischen den beiden Ausgängen ist,
wobei ein Speicherkondensator (22) und eine Rücklaufdiode (24) mit jeder der Vielzahl von Sekundärwicklungen verbunden sind; und
ein Array (112) mit einer Vielzahl von Elektroden, die in elektrischer Kommunikation mit den beiden Ausgängen des Signalverstärkers stehen.

2. Handgerät nach Anspruch 1, wobei das Windungsverhältnis zwischen der Primärwicklung und jeder der Vielzahl von Sekundärwicklungen eins zu eins ist.

3. Handgerät nach Anspruch 1, wobei jede Rücklaufdiode und jeder Speicherkondensator in einer Reihenkonfiguration mit einer entsprechenden der Vielzahl von Sekundärwicklungen gekoppelt sind.

4. Handgerät nach Anspruch 1, wobei die Rücklaufdiode und der Speicherkondensator in einer Parallelkonfiguration mit jeder der Vielzahl von Sekundärwicklungen gekoppelt sind.

5. Handgerät nach Anspruch 4, wobei ein Filterkondensator in einer Parallelkonfiguration mit dem Speicherkondensator gekoppelt ist,
wobei optional ein Ausgleichskondensator in einer Parallelkonfiguration mit jeder der Vielzahl von Sekundärwicklungen gekoppelt ist,
wobei ferner optional ein Thermistor zwischen der Vielzahl von Sekundärwicklungen und der Primärwicklung gekoppelt ist.

6. Handgerät nach Anspruch 1, wobei die Vielzahl von Sekundärwicklungen mindestens fünf Sekundärwicklungen aufweist.

7. Handgerät nach Anspruch 1, wobei die Primärwicklung, die Vielzahl von Sekundärwicklungen, der Speicherkondensator und die Rücklaufdiode jeweils in einem Signalgeneratorgehäuse angeordnet sind.

8. Elektroporationsvorrichtung (104), die Folgendes umfasst:
ein Gehäuse (108);
einen Signalgenerator (32), der innerhalb des Gehäuses positioniert ist, wobei der Signalgenerator Folgendes aufweist:
einen Signalverstärker (5) mit einer Primärwicklung (12) und einer Vielzahl von Sekundärwicklungen (14A-14E), die in einer Reihenkonfiguration miteinander gekoppelt sind, wobei ein Speicherkondensator (22) und eine Rücklaufdiode (24) mit jeder der Vielzahl von Sekundärwicklungen gekoppelt sind und die Vielzahl von Sekundärwicklungen zwei Ausgänge (18A, 18B) aufweist, so dass eine Spannung über der Vielzahl von Sekundärwicklungen gleich der Spannungsdifferenz zwischen den beiden Ausgängen ist;
ein Netzteil (34), und
einen Leistungsschalter (36), der so ausgebildet ist, dass er eine Spannung von dem Netzteil über die Primärwicklung liefert; und
ein Array (112) mit einer oder mehreren Elektroden in elektrischer Kommunikation mit den beiden Ausgängen des Signalgenerators.

9. Elektroporationsvorrichtung nach Anspruch 8, wobei das Windungsverhältnis zwischen der Primärwicklung und jeder der Vielzahl von Sekundärwicklungen eins zu eins ist.

10. Elektroporationsvorrichtung nach Anspruch 8, wobei das Netzteil eine wiederaufladbare Batterie aufweist, wobei die wiederaufladbare Batterie optional eine Lithium-Ionen-Batterie aufweist.

11. Elektroporationsvorrichtung nach Anspruch 8, wobei die Rücklaufdiode und der Speicherkondensator in einer Reihenkonfiguration miteinander gekoppelt sind,
wobei optional die Rücklaufdiode und der Speicherkondensator mit jeder der Vielzahl von Sekundärwicklungen in einer Parallelkonfiguration gekoppelt sind,
wobei ferner optional ein Filterkondensator in Parallelkonfiguration mit dem Speicherkondensator gekoppelt ist.

12. Elektroporationsvorrichtung nach Anspruch 8, wobei ein Ausgleichskondensator mit jeder der Vielzahl von Sekundärwicklungen in einer Parallelkonfiguration gekoppelt ist.

13. Elektroporationsvorrichtung nach Anspruch 8, wobei ein Thermistor zwischen der Vielzahl von Sekundärwicklungen und der Primärwicklung gekoppelt ist.

14. Elektroporationssystem, das Folgendes umfasst:
eine Basisstation (109); und
ein Handgerät (100), das abnehmbar mit der Basisstation gekoppelt ist, wobei das Handgerät Folgendes aufweist:
ein Gehäuse (108),
eine Injektionsanordnung (110) mit einer Injektionsnadel (111) zur Verabreichung eines Wirkstoffs an Zellen eines Zielgewebes,
ein Netzteil (34),
einen Signalgenerator (32), der innerhalb des Gehäuses des Handgerätes positioniert ist und in betriebsfähiger Kommunikation mit der Injektionsanordnung steht, wobei der Signalgenerator Folgendes umfasst:
einen Signalverstärker (5) mit einer Primärwicklung (12) und einer Vielzahl von Sekundärwicklungen (14A-14E), die in einer Reihenkonfiguration miteinander gekoppelt sind, wobei ein Speicherkondensator (22) und eine Rücklaufdiode (24) mit jeder der Vielzahl von Sekundärwicklungen gekoppelt sind und die Vielzahl von Sekundärwicklungen zwei Ausgänge (18A, 18B) aufweist, so dass eine Spannung über der Vielzahl von Sekundärwicklungen gleich der Spannungsdifferenz zwischen den beiden Ausgängen ist, und einen Leistungsschalter (36), der so ausgebildet ist, dass er eine Spannung von dem Netzteil über die Primärwicklung liefert, und
ein Array (112) mit mindestens einer Elektrode (111), die sich von einem vorderen Ende des Gehäuses nach außen erstreckt und in elektrischer Kommunikation mit den beiden Ausgängen des Signalgenerators steht,
wobei die mindestens eine Elektrode von dem Signalgenerator erzeugte Elektroporationsimpulse an die Zellen abgibt, um reversible Poren in den Zellen zu induzieren, die es dem Wirkstoff ermöglichen, in die Zellen einzudringen.

15. Elektroporationssystem nach Anspruch 14, wobei die Basisstation in elektrischer Kommunikation mit dem Netzteil steht, wenn die Basisstation mit dem Handgerät gekoppelt ist.

## Revendications

1. Combiné (100) destiné à être utilisé dans un dispositif d'électroporation (104), le combiné comprenant :
un logement (108) ;
un amplificateur de signaux (5) positionné à l'intérieur du logement, l'amplificateur de signaux comprenant : un enroulement primaire (12),
une pluralité d'enroulements secondaires (14A-14E) couplés ensemble dans une configuration en série, la pluralité d'enroulements secondaires présentant deux sorties (18A, 18B), de telle sorte qu'une tension aux bornes de la pluralité d'enroulements secondaires soit égale à la différence de tension entre les deux sorties,
dans lequel un condensateur de stockage (22) et une diode de retour (24) sont couplés à chacun de la pluralité d'enroulements secondaires ; et
un réseau (112) présentant une pluralité d'électrodes en communication électrique avec les deux sorties de l'amplificateur de signaux.

2. Combiné selon la revendication 1, dans lequel un rapport de transformation entre l'enroulement primaire et chacun de la pluralité d'enroulements secondaires est de une à une.

3. Combiné selon la revendication 1, dans lequel chaque diode de retour et condensateur de stockage sont couplés à un enroulement respectif de la pluralité d'enroulements secondaires dans une configuration en série.

4. Combiné selon la revendication 1, dans lequel la diode de retour et le condensateur de stockage sont couplés à chacun de la pluralité d'enroulements secondaires dans une configuration parallèle.

5. Combiné selon la revendication 4, dans lequel un condensateur filtrant est couplé dans une configuration parallèle avec le condensateur de stockage, éventuellement dans lequel un condensateur d'équilibrage est couplé à chacun de la pluralité d'enroulements secondaires dans une configuration parallèle, en outre éventuellement dans lequel une thermistance est couplée entre la pluralité d'enroulements secondaires et l'enroulement primaire.

6. Combiné de la revendication 1, dans lequel la pluralité d'enroulements secondaires comprend au moins cinq enroulements secondaires.

7. Combiné selon la revendication 1, dans lequel l'enroulement primaire, la pluralité d'enroulements secondaires, le condensateur de stockage et la diode de retour sont chacun disposés dans un logement de générateur de signaux.

8. Dispositif d'électroporation (104) comprenant :
un logement (108) ;
un générateur de signaux (32) positionné à l'intérieur du logement, le générateur de signaux comprenant :
un amplificateur de signaux (5) présentant un enroulement primaire (12) et une pluralité d'enroulements secondaires (14A-14E) couplés ensemble dans une configuration en série, dans lequel un condensateur de stockage (22) et une diode de retour (24) sont couplés à chacun de la pluralité d'enroulements secondaires, et la pluralité d'enroulements secondaires présentant deux sorties (18A, 18B), de telle sorte qu'une tension aux bornes de la pluralité d'enroulements secondaires soit égale à la différence de tension entre les deux sorties ;
une alimentation électrique (34), et
un commutateur d'alimentation (36) configuré pour fournir une tension à partir de l'alimentation électrique aux bornes de l'enroulement primaire ; et
un réseau (112) présentant une ou plusieurs électrodes en communication électrique avec les deux sorties du générateur de signaux.

9. Dispositif d'électroporation selon la revendication 8, dans lequel un rapport de transformation entre l'enroulement primaire et chacun de la pluralité d'enroulements secondaires est de 1/1.

10. Dispositif d'électroporation selon la revendication 8, dans lequel l'alimentation comprend une batterie rechargeable, éventuellement dans lequel la batterie rechargeable comprend une batterie lithium-ion.

11. Dispositif d'électroporation selon la revendication 8, dans lequel la diode de retour et le condensateur de stockage sont couplés l'un à l'autre dans une configuration en série, éventuellement dans lequel la diode de retour et le condensateur de stockage sont couplés à chacun de la pluralité d'enroulements secondaires dans une configuration parallèle, en outre éventuellement dans lequel un condensateur de filtrage est couplé dans une configuration parallèle au condensateur de stockage.

12. Dispositif d'électroporation selon la revendication 8, dans lequel un condensateur d'équilibrage est couplé à chacun de la pluralité d'enroulements secondaires dans une configuration parallèle.

13. Dispositif d'électroporation selon la revendication 8, dans lequel une thermistance est couplée entre la pluralité d'enroulements secondaires et l'enroulement primaire.

14. Système d'électroporation comprenant :
une station de base (109) ; et
un combiné (100) couplé de manière amovible à la station de base, le combiné comprenant :
un logement (108),
un ensemble d'injection (110) comprenant une aiguille hypodermique (111) pour administrer un agent à des cellules d'un tissu cible,
une alimentation électrique (34),
un générateur de signaux (32) positionné à l'intérieur du logement du combiné et en communication fonctionnelle avec l'ensemble d'injection, le générateur de signaux comprenant :
un amplificateur de signaux (5) présentant un enroulement primaire (12), et une pluralité d'enroulements secondaires (14A-14E) couplés ensemble dans une configuration en série, dans lequel un condensateur de stockage (22) et une diode de retour (24) sont couplés à chacun de la pluralité d'enroulements secondaires, et la pluralité d'enroulements secondaires présentant deux sorties (18A, 18B), de telle sorte qu'une tension aux bornes de la pluralité d'enroulements secondaires soit égale à la différence de tension entre les deux sorties, et
un commutateur d'alimentation (36) configuré pour fournir une tension à partir de l'alimentation aux bornes de l'enroulement primaire, et
un réseau (112) présentant au moins une électrode (111) s'étendant vers l'extérieur à partir d'une extrémité avant du logement et en communication électrique avec les deux sorties du générateur de signaux, dans lequel l'au moins une électrode administre des impulsions d'électroporation générées par le générateur de signaux aux cellules pour induire des pores réversibles dans les cellules permettant à l'agent de passer dans les cellules.

15. Système d'électroporation selon la revendication 14, dans lequel la station de base est en communication électrique avec l'alimentation lorsque la station de base est couplée au combiné.
